# EUROPEAN PATENT APPLICATION

(11) **EP 4 104 818 A1**
(43) Date of publication of application: **21.12.2022**
(21) Application number: 21180145.1
(22) Date of filing: 17.06.2021
(51) Int. Cl.: A61K 9/00, A61K 9/08, A61K 9/107, A61K 47/10, A61K 47/20, A61K 31/00, A61P 1/00

(54) **OPHTHALMIC COMPOSITION COMPRISING XANTHOHUMOL**

(71) Applicant: Skeye Pharma GbR, 80802 München (DE)
(72) Inventor: KASSUMEH, Stefan, 80333 München (DE); OHLMANN, Andreas, 80801 München (DE); PRIGLINGER, Siegfried, 81247 München (DE)
(74) Representative: Vossius & Partner Patentanwälte Rechtsanwälte mbB

(57) **Abstract**

The present invention relates to an ophthalmic composition comprising xanthohumol or a stereoisomer, tautomer, pharmaceutically acceptable salt, solvate or prodrug thereof. The present invention furthermore concerns xanthohumol or a stereoisomer, tautomer, pharmaceutically acceptable salt, solvate or prodrug thereof for use in the treatment of corneal surface disorders, such as dry eye syndrome, or to diagnose, cure, mitigate, treat, or prevent corneal surface disorders, such as dry eye syndrome, in a mammal.

## Description

### Field of Invention

The present invention relates to an ophthalmic composition comprising xanthohumol or a stereoisomer, tautomer, pharmaceutically acceptable salt, solvate or prodrug thereof. The present invention furthermore concerns xanthohumol or a stereoisomer, tautomer, pharmaceutically acceptable salt, solvate or prodrug thereof for use in the treatment of corneal surface disorders, such as dry eye syndrome, or to diagnose, cure, mitigate, treat, or prevent corneal surface disorders, such as dry eye syndrome, in a mammal.

### Background

The Dry Eye Syndrome (DES) is a multifactorial disease of the ocular surface and the tear film. Due to tear film instability, either because of poor secretion or fast evaporation, and subsequent ocular surface inflammation, typical symptoms arise. Namely, foreign body sensation, blurry vision, eye dryness and redness as well as light sensitivity. All of them eventually reduce a patient's quality of life substantially. The prevalence of DES is difficult to estimate. However, rates range from 5% to up to 50% depending on the geographical region, sex, age and comorbidities. Above the age of 40 years, DES rates can be as high as 75% with a tendency of women being affected more frequently. Over the course of the last years, extensive research led to the definition of Dry Eye Syndrome being an inflammatory disease. Stress to the ocular surface either toxic, mechanical, via environmental factors (e.g. ultraviolet light), microbial antigens or endogenous factors can trigger inflammatory mechanisms. All of them eventually promote the secretion of reactive oxygen species (ROS) and thus lead to oxidative stress. This is where the vicious circle of ocular surface damage and inflammation begins. A key pathogenic step towards human dry eye via inflammation and oxidative stress is hyperosmolarity of the tears. It causes damage to corneal epithelial cells by activating cascades of inflammatory events that consecutively release a vast variety of inflammatory mediators into the tears. Therefore, another part of the inflammatory cycle of DES is corneal epithelial integrity.

Currently, depending on the form and severity of the dry eye, a multimodal therapeutic approach is taken. The foundation of all forms and severity grades are artificial tears. They may for example contain hyaluronic acid, cellulose derivatives or hydroxypropyl guar and come in low-viscosity preparations or high-viscosity gels. To break the vicious circle of ocular surface inflammation and corneal epithelial damage either a short-term anti-inflammatory treatment with topical corticosteroids or long-term with cyclosporine A can be necessary. However, current anti-inflammatory treatment may cause adverse effects, namely gastrointestinal and skin disorders, raised intraocular pressure and cataract as well as eye irritation and ocular discomfort.

In view of the above-mentioned prior art, it was an object of the present invention to provide a composition for the treatment of a corneal surface disorder, such as dry eye syndrome, or to diagnose, cure, mitigate, treat, or prevent a corneal surface disorder, such as dry eye syndrome in a mammal, which can avoid the adverse effects associated with state-of-the-art compositions containing conventional anti-inflammatory compositions.

### Summary of the invention

The present invention may be summarized by the following items 1 to 41:
Item 1. An ophthalmic composition comprising xanthohumol or a stereoisomer, tautomer, pharmaceutically acceptable salt, solvate or prodrug thereof.
Item 2. The ophthalmic composition according to item 1, wherein the concentration of xanthohumol or a stereoisomer, tautomer, pharmaceutically acceptable salt, solvate or prodrug thereof in the ophthalmic composition is in a range of from 0.001 to 100 µM, preferably 0.01 to 20 µM, more preferably 0.1 to 10 µM, even more preferably 0.2 to 10 µM, still more preferably 0.5 to 6 µM, most preferably 1 to 3 µM, based on the total volume of the ophthalmic composition.
Item 3. The ophthalmic composition according to item 1 or 2, wherein the ophthalmic composition is an aqueous composition, preferably a solution or an emulsion, such as an oil-in-water emulsion.
Item 4. The ophthalmic composition according to any one of items 1 to 3, wherein the ophthalmic composition has a pH in the range of from 7 to 7.5, preferably 7.2 to 7.4, more preferably about 7.3 or about 7.4.
Item 5. The ophthalmic composition according to any one of items 1 to 4, wherein the ophthalmic composition further comprises a buffer, which is preferably selected from acetate buffers, citrate buffers, phosphate buffers, borate buffers, or a combination thereof, wherein the buffer more preferably contain a phosphate or borate buffer.
Item 6. The ophthalmic composition according to item 5, wherein the buffer is contained in the ophthalmic composition at a concentration in a range of from 1 nM to 100 mM, preferably 1 µM to 1 mM, based on the total volume of the ophthalmic composition.
Item 7. The ophthalmic composition according to any one of items 1 to 6, wherein the ophthalmic composition comprises boric acid at a concentration of 0.02 wt.-% to 2 wt.-%, based on the total weight of the ophthalmic composition.
Item 8. The ophthalmic composition according to any one of items 1 to 7, wherein the ophthalmic composition further comprises one or more preservatives, wherein the one or more preservatives are preferably selected from cationic preservatives such as quaternary ammonium compounds such as benzalkonium chloride or polyquad; guanidine-based preservatives such as polyhexamethylene biguanide (PHMB) or chlorhexidine; chlorobutanol; mercury preservatives such as thimerosal, phenylmercuric acetate or phenylmercuric nitrate; and oxidizing preservatives such as stabilized oxychloro complexes.
Item 9. The ophthalmic composition according to item 8, wherein the ophthalmic composition comprises the one or more preservatives at a concentration in a range of from 0.0001 wt.-% to 25 wt.-%, preferably 0.002 wt.-% to 0.05 wt.-%, more preferably 0.005 wt.-% to 0.02 wt.-%, even more preferably about 0.01 wt.-%, based on the total weight of the ophthalmic composition.
Item 10. The ophthalmic composition according to any one of items 1 to 9, wherein the ophthalmic composition further comprises one or more demulcents, wherein the one or more demulcents preferably include one or more polymers, preferably selected from polyvinyl alcohol, povidone, hydroxypropyl methyl cellulose, poloxamers, carboxymethyl cellulose, hydroxyethyl cellulose and acrylates, more preferably wherein the one or more demulcents include carboxymethylcellulose sodium.
Item 11. The ophthalmic composition according to item 10, wherein the ophthalmic composition comprises the one or more demulcents at a concentration in a range of from 0.1 wt.-% to 2 wt.-%, preferably from 0.3 wt.-% to 0.7 wt.-%, more preferably from 0.3 wt.-% to 0.5 wt.-%, based on the total weight of the ophthalmic composition.
Item 12. The ophthalmic composition according to any one of items 1 to 11, wherein the ophthalmic composition further comprises one or more surfactants, wherein the one or more surfactants preferably include one or more alcohols; amine oxides; block polymers; carboxylated alcohol or alkylphenol ethoxylates; carboxylic acids/fatty acids; ethoxylated alcohols; ethoxylated alkylphenols; ethoxylated arylphenols; ethoxylated fatty acids; ethoxylated fatty esters or oils (animal and vegetable); fatty esters; fatty acid methyl ester ethoxylates; glycerol esters; glycol esters; lanolin-based derivatives; lecithin and lecithin derivatives; lignin and lignin derivatives; methyl esters; monoglycerides and derivatives; polyethylene glycols; polymeric surfactants; propoxylated and ethoxylated fatty acids, alcohols, or alkyl phenols; protein-based surfactants; sarcosine derivatives; sorbitan derivatives; sucrose and glucose esters and derivatives, wherein the one or more surfactants more preferably contain polyoxyethylene (80) sorbitan monooleate.
Item 13. The ophthalmic composition according to item 12, wherein the ophthalmic composition comprises the one or more surfactants at a concentration in a range of from 0.001 wt.-% to 5 wt.-%, preferably from 0.1 wt.-% to 2 wt.-%, more preferably from 0.3 wt.-% to 0.7 wt.-%, even more preferably from 0.3 wt.-% to 0.5 wt.-%, based on the total weight of the ophthalmic composition.
Item 14. The ophthalmic composition according to any one of items 1 to 13, wherein the ophthalmic composition further comprises one or more stabilizers, wherein the one or more stabilizers preferably include one or more selected from polyvinyl alcohol, povidone, hydroxypropyl methyl cellulose, poloxamers, carboxymethyl cellulose, hydroxyethyl cellulose, and acrylate homo- and copolymers.
Item 15. The ophthalmic composition according to item 14, wherein the ophthalmic composition comprises the one or more stabilizers at a concentration in a range of from 0.01 wt.-% to 1 wt.-%, preferably from 0.02% to 0.5 wt.-% or from 0.1 wt.-% to 1 wt.-%, based on the total weight of the ophthalmic composition.
Item 16. The ophthalmic composition according to any one of items 1 to 15, wherein the ophthalmic composition further comprises one or more tonicity agents, wherein the one or more tonicity agents preferably include one or more selected from inorganic salts, preferably alkali metal halides such as sodium chloride or potassium chloride, mannitol and glycerol.
Item 17. The ophthalmic composition according to item 16, wherein the ophthalmic composition comprises the one or more tonicity agents at a concentration in a range of from 0.0001 wt.-% to 5 wt.-%, preferably from 0.2% to 5 wt.-%, more preferably from 0.5 wt.-% to 2 wt.-%, based on the total weight of the ophthalmic composition.
Item 18. The ophthalmic composition according to any one of items 1 to 17, wherein the ophthalmic composition comprises glycerol at a concentration of 0.2 wt.-% to 5 wt.-%, based on the total weight of the ophthalmic composition.
Item 19. The ophthalmic composition according to any one of items 1 to 18, wherein the ophthalmic composition further comprises one or more antioxidants, wherein the one or more antioxidants preferably include one or more selected from sodium metabisulfite, sodium thiosulfate, acetylcysteine, butylated hydroxyanisole and butylated hydroxytoluene.
Item 20. The ophthalmic composition according to item 19, wherein the ophthalmic composition comprises the one or more antioxidants at a concentration in a range of from 0.0001 wt.-% to 5 wt.-%, preferably from 0.2% to 5 wt.-%, more preferably from 0.5 wt.-% to 2 wt.-%, based on the total weight of the ophthalmic composition.
Item 21. The ophthalmic composition according to any one of items 1 to 20, wherein the ophthalmic composition is an emulsion and contains one or more triglycerides, wherein the one or more triglycerides are preferably selected from anise oil, castor oil, clove oil, cassia oil, cinnamon oil, almond oil, corn oil, arachis oil, cottonseed oil, safflower oil, maize oil, linseed oil, rapeseed oil, soybean oil, olive oil, caraway oil, rosemary oil, peanut oil, peppermint oil, sunflower oil, eucalyptus oil and sesame oil, wherein the one or more triglycerides preferably contain castor oil.
Item 22. The ophthalmic composition according to item 21, wherein the ophthalmic composition comprises the one or more triglycerides at a concentration in a range of from 0.0001 wt.-% to 5 wt.-%, preferably from 0.005 wt.-% to 1 wt.-%, more preferably from 0.01 wt.-% to 0.5 wt.-%, based on the total weight of the ophthalmic composition.
Item 23. The ophthalmic composition according to any one of items 1 to 22, wherein the ophthalmic composition is an emulsion and contains one or more ophthalmic acceptable excipients, wherein the one or more ophthalmic acceptable excipients are preferably selected from erythritol, and a carnitine, such as L-carnitine or R-carnitine, wherein the one or more ophthalmic acceptable excipients preferably contain erythritol.
Item 24. The ophthalmic composition according to item 23, wherein the ophthalmic composition comprises the one or more ophthalmic acceptable excipients at a concentration in a range of from 0.05 wt.-% to 5 wt.-%, preferably from 0.1 wt.-% to 0.5 wt.-%, based on the total weight of the ophthalmic composition.
Item 25. The ophthalmic composition according to any one of items 1 to 24, wherein the ophthalmic composition contains one or more antimicrobial agents, wherein the one or more antimicrobial agents are preferably selected from fluoroquinolones, moxifloxacin, ciprofloxacin, gatifloxacin, ofloxacin, besifloxacin, chloramphenicol, B-lactams, cefpodoxime, cefazolin, amoxicillin, amoxiclav, aminoglycosides, tobramycin, amikacin, tetracyclines, doxycycline, macrolides, azithromycin, gentamicin, glycopeptides, vancomycin, acyclovir, gancyclovir, natamycin, fluconazole, voriconazole, amphotericin B, antiprotozoals and metronidazole.
Item 26. The ophthalmic composition according to item 25, wherein the ophthalmic composition comprises the one or more antimicrobial agents at a concentration in a range of from 0.0001 wt.-% to 5 wt.-%, preferably from 0.005 wt.-% to 1 wt.-%, more preferably from 0.1 wt.-% to 1 wt.-%, based on the total weight of the ophthalmic composition.
Item 27. The ophthalmic composition according to any one of items 1 to 26, wherein the ophthalmic composition contains one or more steroids, wherein the one or more steroids are preferably selected from fluorometholon, prednisolon, dexamethasone and loteprednol.
Item 28. The ophthalmic composition according to item 27, wherein the ophthalmic composition comprises the one or more steroids at a concentration in a range of from 0.0001 wt.-% to 5 wt.-%, preferably from 0.005 wt.-% to 1 wt.-%, more preferably from 0.1 wt.-% to 1 wt.-%, based on the total weight of the ophthalmic composition.
Item 29. The ophthalmic composition according to any one of items 1 to 28, wherein the ophthalmic composition contains hyaluronic acid, preferably in an amount of 0.001 wt.-% to 0.05 wt.-%, based on the total weight of the ophthalmic composition.
Item 30. The ophthalmic composition according to any one of items 1 to 27, wherein the balance of the ophthalmic composition is water.
Item 31. The ophthalmic composition according to any one of items 1 to 29, wherein the ophthalmic composition contains one or more organic solvents, wherein the organic solvents are preferably one or more selected from 1-perfluorobutyl-pentane, C₂₋₂₄ alkanols such as ethanol and isopropanol, C₂₋₂₄ alkandiols such as propylene glycol, oligo- or polymers of C₂₋₂₄ alkandiols such as polyethylene glycol, and acetone, wherein the one or more organic solvents preferably have a melting point of 25°C or lower, at a pressure of 1 atm.
Item 32. The ophthalmic composition according to item 31, wherein the ophthalmic composition comprises the one or more organic solvents at a concentration in a range of from 0.01 wt.-% to 50 wt.-%, preferably from 0.01 wt.-% to 5 wt.-%, more preferably from 0.01 wt.-% to 1 wt.-%, based on the total weight of the ophthalmic composition.
Item 33. The ophthalmic composition according to any one of items 1 to 32, wherein ophthalmic composition comprises Astragaloside IV or pharmaceutically acceptable salt, solvate or prodrug thereof, preferably at a concentration in a range of from 0.001 to 100 µM, more preferably 0.01 to 20 µM, even more preferably 0.1 to 10 µM, still more preferably 0.2 to 10 µM, still even more preferably 0.5 to 6 µM, most preferably 1 to 3 µM, based on the total volume of the ophthalmic composition.
Item 34. The ophthalmic composition according to any one of items 1 to 33, wherein the ophthalmic composition is for use in the treatment of a corneal surface disorder, preferably dry eye syndrome, or to diagnose, cure, mitigate, treat, or prevent a corneal surface disorder, preferably dry eye syndrome, in a mammal.
Item 35. Xanthohumol or a stereoisomer, tautomer, pharmaceutically acceptable salt, solvate or prodrug thereof for use in the treatment of a corneal surface disorder, preferably dry eye syndrome, or to diagnose, cure, mitigate, treat, or prevent a corneal surface disorder, preferably dry eye syndrome, in a mammal.
Item 36. A method of treating, diagnosing, curing, mitigating or preventing a corneal surface disorder, preferably dry eye syndrome, comprising administering an effective amount of an opthalmic composition according to any one of items 1 to 33 to an eye of a mammal in need thereof.
Item 37. The ophthalmic composition for use according to item 34, xanthohumol for use according to item 35 or the method according to item 36, wherein the mammal is a human.
Item 38. The ophthalmic composition for use according to item 34 or 37, xanthohumol for use according to item 35 or 37 or the method according to item 36 or 37, wherein xanthohumol is in the form of xanthohumol or a pharmaceutically acceptable salt or solvate thereof.
Item 39. The ophthalmic composition for use according to items 34, 37 and 38, xanthohumol for use according to items 35, 37 and 38 or the method according to items 36, 37 and 38, wherein the dry eye syndrome is lipid anomaly dry eye (LADE), aqueous tear deficiency (ATD), primary mucin anomalies, allergic/toxic dry eye (ADE), primary epitheliopathies and lid surfacing/blinking anomalies (LSADE).
Item 40. The ophthalmic composition for use according to items 34, 37, 38 and 39, xanthohumol for use according to items 35, 37, 38 and 39 or the method according to items 36, 37, 38 and 39, wherein the ophthalmic composition or xanthohumol is to be used before, during or after a surgical treatment of the eye, e.g. refractive surgeries, glaucoma surgeries and/or cataract surgeries.
Item 41. The ophthalmic composition for use according to items 34, 37, 38 and 39, xanthohumol for use according to items 35, 37, 38 and 39 or the method according to items 36, 37, 38 and 39, wherein the ophthalmic composition or xanthohumol is to be used in ocular surface and corneal diseases, such as corneal erosion and keratitis punctata.

### Brief description of the figures

**Figure 1****: Xanthohumol Improves hCEC Viability.** WST-1 assay of hCECs following 72 hours of treatment with DMSO (control) or xanthohumol [XH; 1 µM, 2.5 µM and 5 µM]. **p < 0.01; ****p < 0.0001; n = 16 for all groups; 4 independent experiments.
**Figure 2****: Xanthohumol Does Not Decrease hCEC Proliferation.** BrdU cell proliferation assay of hCECs following 24 hours of treatment with DMSO (control) or xanthohumol [XH; 1 µM, 2.5 µM and 5 µM]. n ≥ 18 for all groups; 6 independent experiments.
**Figure 3****: Xanthohumol Enhances hCEC Migration *In Vitro.*** Cell scratch assay of hCECs with a follow-up of 72 hours under the treatment with DMSO (control) or xanthohumol [XH; 1 µM and 2.5 µM]. *p < 0.05; **p < 0.01; n ≥ 24 for all groups.
**Figure 4****: No Antimigratory Effects of Xanthohumol *Ex Vivo.*** Corneal erosion assay on bovine corneas with a follow-up of 72 hours under the treatment with DMSO (control) or xanthohumol [XH; 1 µM and 2.5 µM]. n ≥ 6 for all groups.
**Figure 5****: Xanthohumol Increases Corneal Epithelial Cell Viability Under Oxidative Stress.** WST-1 assay after incubation of hCECs with DMSO (control), H₂O₂ [100 µM] or H₂O₂ and xanthohumol (XH) in a concentration of 1 µM or 2.5 µM for 24 hours. *p < 0.05; ***p < 0.001, ****p < 0.0001; n ≥ 8 for all groups; 4 independent experiments.

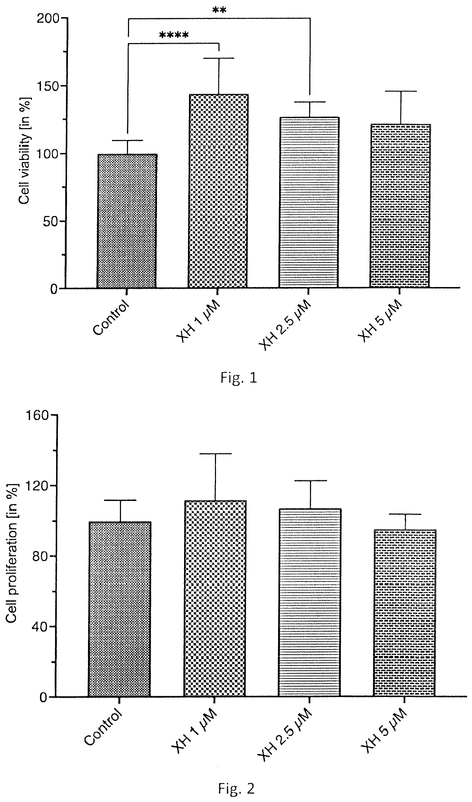

**Figure 1****: Xanthohumol Decreases Intracellular ROS Levels Under Oxidative Stress.** Dihydrorhodamine 123 fluorescence after incubation of hCECs with DMSO (control), H₂O₂ [100 µM] or H₂O₂ and xanthohumol (XH) in a concentration of 1 µM or 2.5 µM for 2 hours. *p < 0.05; n ≥ 8 for all groups; 3 independent experiments.

### Detailed description

The present invention relates to an ophthalmic composition comprising xanthohumol or a stereoisomer, tautomer, pharmaceutically acceptable salt, solvate or prodrug thereof. The present invention furthermore concerns xanthohumol or a stereoisomer, tautomer, pharmaceutically acceptable salt, solvate or prodrug thereof for use in the treatment of a corneal surface disorder, or to diagnose, cure, mitigate, treat, or prevent a corneal surface disorder in a mammal. The corneal surface disorder is preferably dry eye syndrome. Other examples of corneal surface disorder include epithelium erosion.

The term "xanthohumol" as used herein refers to 1-[2,4-Dihydroxy-6-methoxy-3-(3-methyl-but-2-enyl)-phenyl]-3-(4-hydroxyphenyl)-propenon. Xanthohumol may be represented by the following formula:

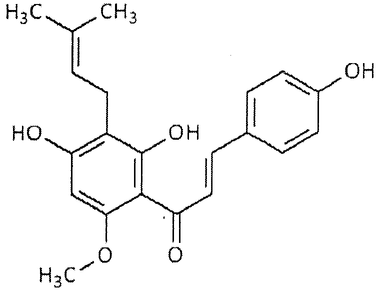

Xanthohumol can be obtained by methods known to the skilled person and is commercially available. In particular, the extraction of hops (*Humulus lupulus*) with organic solvents such as acetone can be used for the production of xanthohumol. A particular method is disclosed in WO 2015/049561 A1, which also mentions a number of further prior art methods for obtaining xanthohumol. Synthetic approaches are known, such as the total synthesis published by Khupse et al. in J. Nat. Prod. 2007, 70, 9, 1507-1509. However, the extraction from hops is typically the most economic approach.

The concentration of xanthohumol or a stereoisomer, tautomer, pharmaceutically acceptable salt, solvate or prodrug thereof in the ophthalmic composition is typically in a range of from 0.001 to 100 µM, preferably 0.01 to 20 µM, more preferably 0.1 to 10 µM, even more preferably 0.2 to 10 µM, still more preferably 0.5 to 6 µM, most preferably 1 to 3 µM, based on the total volume of the ophthalmic composition.

The ophthalmic composition is preferably an aqueous composition. The term "aqueous", when characterizing the compositions of the invention, means that the compositions comprise water, preferable at least 1 wt% of water with respect to the total weight of the composition, more preferably at least 10 wt% of water, more preferably at least 20 wt% of water, more preferably at least 30 wt% of water, more preferably at least 40 wt% of water, more preferably at least 50 wt% of water, more preferably at least 60 wt% of water, more preferably at least 70 wt% of water, more preferably at least 80 wt% of water, more preferably at least 85 wt% of water, more preferably at least 90 wt% of water. It is particularly preferred that the ophthalmic composition of the present invention comprises at least 80 wt% of water with respect to the total weight of the composition.

The ophthalmic composition is preferably a solution. The term "solution" as used herein preferably indicates that even after leaving the ophthalmic composition standing for one week, no sediments or precipitates are formed. More preferably, the term "solution" as used herein indicates that the ophthalmic composition can be passed through a filter having a pore size of about 0.22 µm made of polyvinylidene difluoride (PVDF) (such as a Fluorodyne^{®} II-Filter) without leaving a residue on the filter.

Even more preferably, the ophthalmic composition of the present invention is an emulsion, such as an oil-in-water emulsion. Suitable approaches for forming ophthalmic emulsions are known to the skilled person and, e.g. described in WO 2006/076308 A1, WO 2010/141588 A1 and WO 2011/068955 A1. As set out in the latter publication, an ophthalmic emulsion typically includes the water forming an aqueous phase, oil forming an oil phase, a hydrophilic surfactant, a hydrophobic surfactant, a charged phospholipid, borate; and a mucoadhesive polymer such as a galactomannan polymer. Accordingly, the hydrophilic surfactant is typically present in the emulsion in an amount that is at least about 0.01 wt.-%, more typically at least about 0.08 wt.-% and even more typically at least about 0.14 wt.-%. The hydrophilic surfactant is typically present in the emulsion in an amount that is no greater than about 1.5 wt.-%, more typically no greater than about 0.8 wt.-% and even more typically no greater than about 0.44 wt.-%. The hydrophilic surfactant can be a fatty acid, an ester, an ether, an acid or any combination thereof. The hydrophilic surfactant may be ionic or non-ionic, but is preferably non-ionic. Many suitable surfactants/emulsifiers are nonionic ester or ether emulsifiers comprising a polyoxyalkylene moiety, especially a polyoxyethylene moiety, often containing from about 2 to 80, and especially 5 to 60 oxyethylene units, and/or contain a polyhydroxy compound such as glycerol or sorbitol or other alditols as hydrophilic moiety. The hydrophilic moiety can contain polyoxypropylene. The emulsifiers additionally contain a hydrophobic alkyl, alkenyl or aralkyl moiety, normally containing from about 8 to 50 carbons and particularly from 10 to 30 carbons. Examples of hydrophilic surfactants/emulsifiers include ceteareth-10-25, ceteth-10-25, steareth-10-25, and PEG-15-25 stearate or distearate. Other suitable examples include C₁₀₋₂₀ fatty acid mono, di or tri- glycerides. Further examples include C₁₈₋₂₂ fatty alcohol ethers of polyethylene oxides (8 to 12 EO units). One particularly preferred hydrophilic surfactant is polyoxyethylene-40-stearate, which is sold under the tradename MYRJ-52, which is commercially available from Nikko Chemicals. The hydrophobic surfactant is typically present in the emulsion in an amount that is at least about 0.01 wt.-%, more typically at least about 0.1 wt.-% and even more typically at least about 0.16 wt.-%. The hydrophobic surfactant is typically present in the emulsion in an amount that is no greater than about 10.0 wt.-%, more typically no greater than about 2.0 wt.-% and even more typically no greater than about 0.62 wt.-%. The hydrophobic surfactant can be a fatty acid, an ester, an ether, an acid or any combination thereof. The hydrophobic surfactant may be ionic or non-ionic, but is preferably non-ionic. The hydrophobic surfactant will typically include a hydrophobic moiety. The hydrophobic moiety can be either linear or branched and is often saturated, though it can be unsaturated, and is optionally fluorinated. The hydrophobic moiety can comprise a mixture of chain lengths, for example those deriving from tallow, lard, palm oil sunflower seed oil or soya bean oil. Such non- ionic surfactants can also be derived from a polyhydroxy compound such as glycerol or sorbitol or other alditols. Examples of hydrophobic surfactants include, without limitation, sorbitan fatty acid esters such as sorbitan monoleate, sorbitan monostearate, sorbitan monolaurate, sorbitan monopalmitate, sorbitan monoisostearate, sorbitan trioleate, sorbitan tristearate, sorbitan sesquioleate, sorbitan sesquistearate, combinations thereof or the like. One particularly preferred hydrophobic surfactant is a sorbitan tristearate sold under the tradename SPAN-65, which is commercially available from Croda Worldwide.

As set out in WO 2011/068955 A1 the types of galactomannans that may be used are typically derived from guar gum, locust bean gum and tara gum. As used herein, the term "galactomannan" refers to polysaccharides derived from the above natural gums or similar natural or synthetic gums containing mannose or galactose moieties, or both groups, as the main structural components. Preferred galactomannans of the present invention are made up of linear chains of (1-4)-beta-D-mannopyranosyl units with alpha-D-galactopyranosyl units attached by (1-6) linkages. With the preferred galactomannans, the ratio of D-galactose to D-mannose varies, but generally will be from about 1:2 to 1:4. Galactomannans having a D-galactose:D-mannose ratio of about 1:2 are most preferred. Additionally, other chemically modified variations of the polysaccharides are also included in the "galactomannan" definition. For example, hydroxyethyl, hydroxypropyl and carboxymethylhydroxypropyl substitutions may be made to the galactomannans of the present invention. Non-ionic variations to the galactomannans, such as those containing alkoxy and alkyl (C₁₋₆) groups are particularly preferred when a soft gel is desired (e.g., hydroxylpropyl substitutions). Substitutions in the non-cis hydroxyl positions are most preferred. An example of non-ionic substitution of a galactomannan of the present invention is hydroxypropyl guar, with a molar substitution of about 0.4. Anionic substitutions may also be made to the galactomannans. Anionic substitution is particularly preferred when strongly responsive gels are desired. A galactomannan is typically present in a formulation of the present invention at a concentration of at least about 0.005 wt.-% based on the ophthalmic composition, more typically at least about 0.01 wt.-% based on the ophthalmic composition and even more typically at least about 0.03 wt.-% based on the ophthalmic composition, but typically no greater than about 5 wt.-% based on the ophthalmic composition, more typically no greater than about 1.0 wt.-% based on the ophthalmic composition, still more typically no greater than about 0.3 wt.-% based on the ophthalmic composition and even still more typically no greater than about 0.08 wt.-% based on the ophthalmic composition. Preferred galactomannans of the present invention are guar and hydroxypropyl guar.

As set out in WO 2011/068955 A1, one or more phospholipids are used for aiding in maintaining the stability of the emulsion and for reducing droplet size of the oil. It is known that complex phospholipids can contain a polar group at one end of their molecular structure and a non-polar group at the opposite end of their molecular structure. A discussion of phospholipids can be found in Lehninger, Biochemistry, 2 ed., Worth Publishers, New York, pp. 279-306, incorporated herein by reference for all purposes. Many complex phospholipids are known to the art. They differ in size, shape and the electric charge of their polar head groups. Phosphoglycerides are compounds where one primary hydroxyl group of glycerol is esterified to phosphoric acid, and the other two hydroxyl groups are esterified with fatty acids. The parent compound of the series is, therefore, the phosphoric acid ester of glycerol. This compound has an asymmetric carbon atom and, therefore, the term phosphoglycerides includes stereoisomers. All phosphoglycerides have a negative charge at the phosphate group at pH 7, and the pKa of this group is in the range of 1 to 2. The head groups of phosphatidylinositol, phosphatidylglycerol including diphosphatidylglycerols (having the common name cardiolipins) and the phosphatidylsugars have no electric charge, and all are polar because of their high hydroxyl group content. Because of the negative charge of the phosphate group and the absence of a charge in the head group, the net charge of each of these materials is negative, and these materials are within the scope of the invention. Suitable phospholipids are those carrying a net positive or negative charge under conditions of use. The preferred materials are those carrying a net negative charge because the negatively charged material will be repelled by the negatively charged ocular surface thereby permitting the maintenance of a relatively thick aqueous layer upon application to the eye. The most preferred phospholipid is an anionic phospholipid named dimyristoyl phosphatidylglycerol (DMPG), which is a polyol with a net negative charge, Phosphatidylglycerol or a phosphatidylinositol are other examples. Suitable phospholipid additives are disclosed in the above cited U.S. Patent No. 4,914,088, which is fully incorporated herein by reference for all purposes. Most phospholipids are water insoluble. However, for application to the eye, it is desirable that the phospholipid be homogeneously distributed throughout an aqueous medium. For those few phospholipids having a solubility within a useful concentration range for use as a treatment composition, a simple aqueous solution of the phospholipid in saline is satisfactory. For those phospholipids that are essentially water insoluble, an aqueous composition in the form of an emulsion may be used. An emulsion provides a treatment composition where the phase containing the phospholipid component is homogeneously distributed throughout the aqueous vehicle. A clinically practical concentration range for the phospholipid in its vehicle varies from about 0.05 to 7.0 wt.-% phospholipid by weight, and more preferably varies from about 0.1 and 5.0 wt.-%. It should be noted that the most desired concentration for the phospholipid in the aqueous composition will vary from subject to subject.

The normal physiological pH of the ocular surface in humans is on average generally about 7.1. It is therefore preferred that the ophthalmic composition has a pH in the range of from 7 to 7.5, preferably 7.2 to 7.4, more preferably about 7.3 or about 7.4. As a skilled person will understand, the pH of an ophthalmic composition is preferably stabilized by including one or more buffers, which are preferably selected from acetate buffers, citrate buffers, phosphate buffers, borate buffers, or a combination thereof. In the present invention, it is further preferred that the buffer contains or consists of a phosphate buffer. The concentration of the compounds acting as buffers in the composition of the invention may be adjusted by the skilled person based on their common general knowledge. Typically, the buffer is contained in the ophthalmic composition at a concentration in a range of from 1 nM to 100 mM, preferably 1 µM to 1 mM, based on the total volume of the ophthalmic composition.

Suitable buffer solutions may be prepared following well-known procedures. For example, in order to obtain a 0.1 M phosphate puffer with a pH of 7.4, 20.214 g of Na₂HPO₄^{∗}7H₂O and 3.394 g of NaH₂PO₄^{∗}H₂O are added to 800 mL of distilled water, the pH of the solution is adjusted to a pH of 7.4 using diluted HCI or diluted NaOH, followed by addition of distilled water until the volume is 1 liter.

Examples of pH adjusting agent may by selected from lactic acid and salts thereof (such as sodium lactate, potassium lactate and calcium lactate), citric acid and salts thereof (such as sodium citrate, potassium citrate, calcium citrate and lithium citrate), tartaric acid and salts thereof (such as sodium tartrate potassium tartrate, calcium tartrate and lithium tartrate), acetic acid and salts thereof (such as sodium acetate, potassium acetate and calcium acetate), hydrochloric acid, boric acid and salts thereof (sodium borate), sulphuric acid and salts thereof (such as sodium sulphate and potassium sulphate), nitric acid, hydrochloric acid, phosphoric acid and salts thereof (such as sodium dihydrogen phosphate, sodium monohydrogen phosphate, potassium dihydrogen phosphate lithium phosphate, potassium phosphate and calcium phosphate), carbonic acid and salts thereof (such as sodium carbonate, sodium hydrogen carbonate and potassium hydrogen carbonate), maleic acid and salts thereof (lithium maleate, sodium maleate, potassium maleate and calcium maleate), succinic acid and salts thereof (lithium succinate, sodium succinate, potassium succinate and calcium succinate), sodium hydroxide, potassium hydroxide, triethanolamine, diisopropanolamine, ammonia, tris(hydroxymethyl)aminomethane, tris(hydroxymethyl)aminomethane hydrochloride, and mixtures thereof.

As set out above, phosphate buffers are preferred in the present invention. A further preferred type of puffer is borate buffer, which may be used instead of, or in addition to the phosphate buffer. Accordingly, the ophthalmic composition may comprise boric acid, preferably at a concentration of 0.02 wt.-% to 2 wt.-%, based on the total weight of the ophthalmic composition. Boric acid and its salts are used in many ophthalmic products as a buffer or for isotonisation. It is well-known in the production of eye drops. It has good compatibility with other excipients, with drug substances, and with a wide range of packaging materials.

Preservatives can be an important component of ophthalmic compositions. They provide antimicrobial activity in the packaging and might also prevent decomposition of the active drug. The most common preservatives in ophthalmic compositions are benzalkonium chloride (BAK), chlorobutanol, sodium perborate, and stabilized oxychloro complex (SOC). While it is preferable that the ophthalmic composition of the present invention is (substantially) free of preservative(s), there are certain embodiments where the use of a preservative is desirable (e.g. in certain multiple-dose-formulations). Thus, in certain embodiments, the ophthalmic composition of the present invention preferably comprises one or more preservatives, wherein the one or more preservatives are preferably selected from cationic preservatives such as quaternary ammonium compounds such as benzalkonium chloride or polyquad; guanidine-based preservatives such as polyhexamethylene biguanide (PHMB) or chlorhexidine; chlorobutanol; mercury preservatives such as thimerosal, phenylmercuric acetate or phenylmercuric nitrate; and oxidizing preservatives such as stabilized oxychloro complexes.

The ophthalmic composition of the present invention comprises the one or more preservatives typically at a concentration in a range of from 0.0001 wt.-% to 25 wt.-%, preferably 0.002 wt.-% to 0.05 wt.-%, more preferably 0.005 wt.-% to 0.02 wt.-%, even more preferably about 0.01 wt.-%, based on the total weight of the ophthalmic composition.

The ophthalmic composition may further comprise one or more demulcents. Demulcents (or humectants) are typically included for lubricating mucous membrane surfaces and for relieving dryness and irritation. The term "demulcent", as used herein is intended to mean an agent, usually a water-soluble polymer, which is applied topically to the eye to protect and lubricate mucous membrane surfaces and relieve dryness and irritation. Typical classes of demulcents include
(a) cellulose derivatives such as carboxymethyl cellulose sodium (typically 0.2 to 2.5 wt.-% based on the ophthalmic composition), hydroxyethyl cellulose (typically 0.2 to 2.5 wt.-% based on the ophthalmic composition), hypromellose (typically 0.2 to 2.5 wt.-% based on the ophthalmic composition), or methyl cellulose (typically 0.2 to 2.5 wt.-% based on the ophthalmic composition);
(b) Dextran 70;
(c) Gelatine;
(d) Polyols such as glycerine (typically 0.2 to 1 wt.-% based on the ophthalmic composition), polyethylene glycol 300 (typically 0.2 to 1 wt.-% based on the ophthalmic composition), polyethylene glycol 400 (typically 0.2 to 1 wt.-% based on the ophthalmic composition), polysorbate 80 (typically 0.2 to 1 wt.-% based on the ophthalmic composition), propylene glycol (typically 0.2 to 1 wt.-% based on the ophthalmic composition).
(e) Polyvinyl alcohol (typically 0.1 to 4 wt.-% based on the ophthalmic composition).
(f) Povidone (typically 0.1 to 2 wt.-% based on the ophthalmic composition),

The one or more demulcents used in the present invention preferably include one or more polymers, preferably selected from polyvinyl alcohol, povidone, hydroxypropyl methyl cellulose, poloxamers, carboxymethyl cellulose, hydroxyethyl cellulose and acrylates. More preferably the one or more demulcents include (or consist of) carboxymethylcellulose sodium.

The concentration of the one or more demulcents in the ophthalmic composition of the present invention is typically in a range of from 0.1 wt.-% to 2 wt.-%, preferably from 0.3 wt.-% to 0.7 wt.-%, more preferably from 0.3 wt.-% to 0.5 wt.-%, based on the total weight of the ophthalmic composition.

The ophthalmic composition may further comprise one or more surfactants. The surfactant may be selected from the group consisting of non-ionic surfactants, cationic surfactants, anionic surfactants, zwitterionic surfactants and mixtures of two or more thereof. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adduct containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof. Suitable non-ionic surfactants include poloxamers, tyloxapol, polysorbates polyoxyethylene castor oil derivatives, sorbitan esters, and mixtures of two or more thereof. Suitable cationic surfactants include hexadecyl trimethyl ammonium bromide, CTAB and mixtures of two or more thereof. Suitable anionic surfactants include sodium lauryl ether sulphate (SLES), sodium lauryl sulphate and mixtures of two or more thereof. Suitable zwitterionic surfactants include such as phospholipids, for example lecithin, dipalmitoylphosphatidylcholine (DpPC) and mixtures of two or more thereof.

In the present invention, the one or more surfactants preferably include one or more alcohols; amine oxides; block polymers; carboxylated alcohol or alkylphenol ethoxylates; carboxylic acids/fatty acids; ethoxylated alcohols; ethoxylated alkylphenols; ethoxylated arylphenols; ethoxylated fatty acids; ethoxylated fatty esters or oils (animal and vegetable); fatty esters; fatty acid methyl ester ethoxylates; glycerol esters; glycol esters; lanolin-based derivatives; lecithin and lecithin derivatives; lignin and lignin derivatives; methyl esters; monoglycerides and derivatives; polyethylene glycols; polymeric surfactants; propoxylated and ethoxylated fatty acids, alcohols, or alkyl phenols; protein-based surfactants; sarcosine derivatives; sorbitan derivatives; sucrose and glucose esters and derivatives, wherein the one or more surfactants more preferably contain polyoxyethylene (80) sorbitan monooleate.

Examples of classes of surfactants which are particularly useful in the present invention include: polyethylene glycol sorbitan fatty acid esters (TWEENs, (polyoxyethylene (20) monolaurate), for example TWEEN 20, TWEEN 60 (polyoxyethylene (20) monostearate), TWEEN 80 (polyoxyethylene (20) monooleate)); sorbitan fatty acid esters (SPANs, for example SPAN 20 (sorbitan monolaurate), SPAN 40 (sorbitan monopalmitate), SPAN 80 (sorbitan monooleate)); polyethylene glycol fatty acid ethers (BRIJs, for example BRIJ 35 (polyoxyethylene (20) lauryl ether), BRIJ 58(polyoxyethylene (20) cetyl ether)); polyethylene glycol stearate esters (MRYJs, for example MYRJ S40 polyoxyethylene (40) stearate, MYRJ S50 polyoxyethylene (50) stearate,); polyoxyethylene glycol-block-polypropylene glycol-block-polyoxyethylene glycol-block (Poloxamers, such as polyoxyethylene glycol (80)-polypropylene glycol (27)-polyoxyethylene glycol (80) (Poloxamer 188) and polyoxyethylene glycol (101)-polypropylene glycol (56)-polyoxyethylene glycol (101) (poloxamer 407)) polyethylene glycol lauryl esters (Laureths, for example polyethylene glycol (4) lauryl ester (Laureth 4) and polyethylene glycol (23) lauryl ester (Laureth 23)) and mixtures of two or more thereof. One or more of each type of surfactant may be present in the composition. Mixtures of different types of surfactant may be present in the ophthalmic composition or the present invention. One of the reasons these surfactants are particularly preferred is because of their low irritation potential.

It is preferred that the ophthalmic composition comprises the one or more surfactants at a concentration in a range of from 0.001 wt.-% to 5 wt.-%, preferably from 0.1 wt.-% to 2 wt.-%, more preferably from 0.3 wt.-% to 0.7 wt.-%, even more preferably from 0.3 wt.-% to 0.5 wt.-%, based on the total weight of the ophthalmic composition. It may be preferable to keep the concentration of the one or more surfactants at less than 0.25 wt.-% , more preferably at less than 0.1 wt.-% of the total weight of the ophthalmic composition. The ophthalmic compositions of the present invention may comprise less than 0.075 wt.-% or less than 0.05 wt.-% or 0.01 wt.-% of surfactant based on the total weight of the ophthalmic composition.

The ophthalmic composition of the present invention may further comprise a cosurfactant selected from the group consisting of alcohols, such as ethanol, isopropanol, n-butanol, isobutanol, 2-pentanol, isopentanol, n-pentanol, n-hexanol, 1-decanol; glycols, such as propylene glycol, 1,2-octanediol, tetraglycol, 1,2-hexandiol, polyethylene glycol; C₆₋₁₄ fatty acids or salts thereof, such as sodium laurate; amines or ether-alcohols, such as diethylene glycol monoethyl ether.

The ophthalmic composition may further comprise one or more stabilizers. The one or more stabilizers preferably include one or more selected from polyvinyl alcohol, povidone, hydroxypropyl methyl cellulose, poloxamers, carboxymethyl cellulose, hydroxyethyl cellulose, and acrylate homo- and copolymers. The ophthalmic composition comprises the one or more stabilizers preferably at a concentration in a range of from 0.01 wt.-% to 1 wt.-%, preferably from 0.02% to 0.5 wt.-% or from 0.1 wt.-% to 1 wt.-%, based on the total weight of the ophthalmic composition.

In addition, the ophthalmic composition preferably comprises one or more tonicity agents. Preferable tonicity agents include one or more selected from inorganic salts, preferably alkali metal halides such as sodium chloride or potassium chloride, mannitol and glycerol. More preferred tonicity agents include one or more selected from inorganic salts, most preferred alkali metal halides such as sodium chloride.or potassium chloride.

The ophthalmic composition comprises the one or more tonicity agents preferably at a concentration in a range of from 0.0001 wt.-% to 5 wt.-%, preferably from 0.2% to 5 wt.-%, more preferably from 0.5 wt.-% to 2 wt.-%, based on the total weight of the ophthalmic composition.

It is preferred that the ophthalmic composition has an osmolality of about 100 mOsm/kg to about 600 mOsm/kg, more preferably an osmolality of about 200 mOsm/kg to about 500 mOsm/kg, even more preferably an osmolality of about 250 mOsm/kg to about 350 mOsm/kg, even more preferably an osmolality of about 270 mOsm/kg to about 330 mOsm/kg, yet even more preferably an osmolality of about 280 mOsm/kg to about 310 mOsm/kg. Moreover, the ophthalmic composition is preferably rendered isotonic (e.g., to any of the aforementioned osmolality ranges or values), using one or more alkali metal halides, e.g., sodium chloride.

The ophthalmic composition may comprise glycerol, preferably at a concentration of 0.2 wt.-% to 5 wt.-%, based on the total weight of the ophthalmic composition.

In addition, it is preferred that the ophthalmic composition further comprises one or more antioxidants. Exemplary antioxidants for ophthalmic compositions may include, but are not limited to, zeaxanthin, lutein, canthaxantin, β-carotene, astaxanthin, ascorbic acid or vitamin C, phenolic acids, sorbic acid, sodium bisulfite, sodium metabisulfite, sodium thiosulfate, acetyl cysteine, sodium thiosulfate, ethylene diamine tetraacetic acid (EDTA), sodium nitrite, ascorbyl stearate, ascorbyl palmitate, alpha-thioglycerol, erythorbic acid, cysteine hydrochloride, citric acid, tocopherol or vitamin E, tocopherol acetate, dibutylhydroxytoluene, soybean lecithin, sodium thioglycolate, butylhydroxyanisole, propyl gallate, uric acid, melatonin, thiourea, or salts or combinations thereof.

The one or more antioxidants preferably include one or more selected from sodium metabisulfite, sodium thiosulfate, acetylcysteine, butylated hydroxyanisole and butylated hydroxytoluene.

The one or more antioxidants are preferably contained in the ophthalmic composition of the present invention at a concentration in a range of from 0.0001 wt.-% to 5 wt.-%, preferably from 0.2% to 5 wt.-%, more preferably from 0.5 wt.-% to 2 wt.-%, based on the total weight of the ophthalmic composition.

The ophthalmic composition may preferably comprise Astragaloside IV. It is to be understood that Astragaloside IV may also be used as a pharmaceutically acceptable salt, solvate or prodrug thereof. Astragaloside IV is commercially available under the CAS-No. 84687-43-4 ((2R,3R,4S,5S,6R)-2-(((2aR,3R,4R,5aS,7R,11aR)-4-hydroxy-3-((2R,5S)-5-(2-hydroxypropan-2-yl)-2-methyltetrahydrofuran-2-yl)-2a,5a,8,8-tetramethyl-9-(((2S,3R,4S,5R)-3,4,5-trihydroxytetrahydro-2H-pyran-2-yl)oxy)hexadecahydrocyclopenta[a]cyclopropa[e]phenanthren-7-yl)oxy)-6-(hydroxymethyl)tetrahydro-2H-pyran-3,4,5-triol) and may be represented by the following formula:

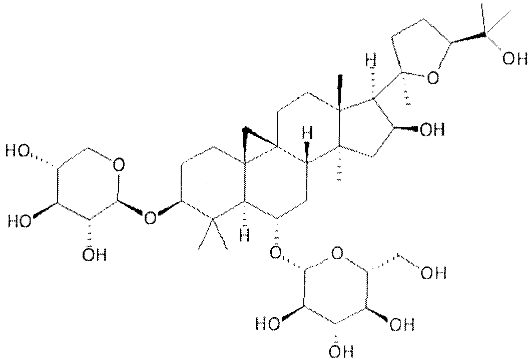

The Astragaloside IV or pharmaceutically acceptable salt, solvate or prodrug thereof is preferably contained at a concentration in a range of from 0.001 to 100 µM, preferably 0.01 to 20 µM, more preferably 0.1 to 10 µM, even more preferably 0.2 to 10 µM, still more preferably 0.5 to 6 µM, most preferably 1 to 3 µM, based on the total volume of the ophthalmic composition. More preferred is, that the range of the concentration of Astragaloside IV or a pharmaceutically acceptable salt, solvate or prodrug thereof in the ophthalmic composition is in a range of from 0.001 to less than 5 µM, preferably in a range of from 0.001 to 4 µM, even more preferably in a range of from 0.01 to 3 µM, based on the total volume of the ophthalmic composition.

The ophthalmic composition is an emulsion and/or contains one or more triglycerides. The one or more triglycerides are preferably selected from anise oil, castor oil, clove oil, cassia oil, cinnamon oil, almond oil, corn oil, arachis oil, cottonseed oil, safflower oil, maize oil, linseed oil, rapeseed oil, soybean oil, olive oil, caraway oil, rosemary oil, peanut oil, peppermint oil, sunflower oil, eucalyptus oil and sesame oil, wherein the one or more triglycerides preferably contain castor oil. In particular, a mixture of castor oil and triglycerides of C₁₀₋₂₀ (preferably C₁₂₋₁₅) fatty acids promotes mucin expression and re-epithelization, in particular in corneal wounds. The term "castor oil" refers to a vegetable oil obtained by pressing the seeds of the castor oil plant (Ricinus communis). It is a triglyceride in which approximately 90% of fatty acid chains are ricinoleate; oleate and linoleates are the other significant components. The average composition of fatty acid chains in castor oil is preferably 85 mol-% to 95 mol-% of ricinoleic acid, 2 mol-% to 6 mol-% of oleic acid, 1 mol-% to 7 mol-% of linoleic acid, 0 mol-% to 1 mol-% of linolenic acid, 0 mol-% to 2.5 mol-% of stearic acid, 0.1 mol-% to 2 mol-% of palmitic acid and 0.2 mol-% to 1.0 mol-% of other fatty acids. The term fatty acid as used herein generally refers to any saturated or unsaturated (e.g. containing 1, 2, 3 or 4 C=C double bonds) C₆₋₃₀-alkanoic acid esters.

The ophthalmic composition of the present invention comprises the one or more triglycerides typically at a concentration in a range of from 0.0001 wt.-% to 5 wt.-%, preferably from 0.005 wt.-% to 1 wt.-%, more preferably from 0.01 wt.-% to 0.5 wt.-%, based on the total weight of the ophthalmic composition.

The ophthalmic composition is furthermore preferably an emulsion and/or contains one or more ophthalmic acceptable excipients. Ophthalmic acceptable excipients are, for example, listed in the Handbook of Pharmaceutical Excipients, 3rd edition (2000), edited by Arthur H. Kibbe, published by American Pharmaceutical Association and Pharmaceutical Press.

In the present invention, the one or more ophthalmic acceptable excipients are preferably selected from erythritol, and a carnitine, such as L-carnitine or R-carnitine, wherein the one or more ophthalmic acceptable excipients preferably contain erythritol. The one or more ophthalmic acceptable excipients are preferably comprised at a concentration in a range of from 0.05 wt.-% to 5 wt.-%, preferably from 0.1 wt.-% to 0.5 wt.-%, based on the total weight of the ophthalmic composition.

The ophthalmic composition of the present invention is preferably sterilized by filtration, i.e. is sterile-filtered. Filtration involves the use of filter material having pores small enough to retain micro-organisms. The micro-organisms are retained because of the small size of the filter pores and partly by adsorption on the walls of pores while passing through the filter. Typical filters suitable for this purpose exhibit, e.g., a pore size of about 0.22 µm and are preferably made of polyvinylidene difluoride (PVDF), polyethersulfone (PES) or nylon. In particular, filters such as Fluorodyne^{®} II-Filter, Supor ^{®} Filter (PES), Ultipor Nylon Filter, which are available from Pall GmbH, Germany, may be used.

The ophthalmic composition may contain one or more antimicrobial agents. In the present invention, the one or more antimicrobial agents are preferably selected from fluoroquinolones, moxifloxacin, ciprofloxacin, gatifloxacin, ofloxacin, besifloxacin, chloramphenicol, B-lactams, cefpodoxime, cefazolin, amoxicillin, amoxiclav, aminoglycosides, tobramycin, amikacin, tetracyclines, doxycycline, macrolides, azithromycin, gentamicin, glycopeptides, vancomycin, acyclovir, gancyclovir, natamycin, fluconazole, voriconazole, amphotericin B, antiprotozoals and metronidazole. The ophthalmic composition comprises the one or more antimicrobial agents typically at a concentration in a range of from 0.0001 wt.-% to 5 wt.-%, preferably from 0.005 wt.-% to 1 wt.-%, more preferably from 0.1 wt.-% to 1 wt.-%, based on the total weight of the ophthalmic composition. Generally, it is preferred that the concentration of the one or more antimicrobial agents is 1 wt.-% or less, more preferably 0.5 wt.-% or less, even more preferably 0.2 wt.-% or less, still more preferably 0.1 wt.-% or less, based on the total weight of the ophthalmic composition.

The ophthalmic composition may furthermore contain one or more steroids. Typical stereoids for ophthalmic compositions include prednisolone, loteprednol, difluprednate, dexamethasone, fluocinolone, fluorometholone, triamcinolone and rimexolone. In the present invention, the one or more steroids are preferably selected from fluorometholon, prednisolone, dexamethasone and loteprednol. The ophthalmic composition may comprise the one or more steroids, e.g., at a concentration in a range of from 0.0001 wt.-% to 5 wt.-%, preferably from 0.005 wt.-% to 1 wt.-%, more preferably from 0.1 wt.-% to 1 wt.-%, based on the total weight of the ophthalmic composition.

It is preferred that the ophthalmic composition contains hyaluronic acid, preferably in an amount of 0.001 wt.-% to 0.05 wt.-%, based on the total weight of the ophthalmic composition.

Generally, it is preferred that the balance of the ophthalmic composition is water. However, in particular when the ophthalmic composition is a solution, the ophthalmic composition may also contain one or more organic solvents. Such organic solvents are preferably one or more selected from 1-perfluorobutyl-pentane, C₂₋₂₄ alkanols such as ethanol and isopropanol, C₂₋₂₄ alkandiols such as propylene glycol, oligo- or polymers of C₂₋₂₄ alkandiols such as polyethylene glycol, and acetone. It is to be understood that the one or more organic solvents preferably have a melting point of 25°C or lower, at a pressure of 1 atm. The one or more organic solvents, if present, are preferably contained at a concentration in a range of from 0.01 wt.-% to 50 wt.-%, preferably from 0.01 wt.-% to 5 wt.-%, more preferably from 0.01 wt.-% to 1 wt.-%, based on the total weight of the ophthalmic composition. Generally, it is preferred that the concentration of the one or more antimicrobial solvents is 1 wt.-% or less, more preferably 0.5 wt.-% or less, even more preferably 0.2 wt.-% or less, based on the total weight of the ophthalmic composition.

### Use of the ophthalmic composition

The ophthalmic composition of the present invention is preferably for use in the treatment of a corneal surface disorder, preferably dry eye syndrome, or to diagnose, cure, mitigate, treat, or prevent a corneal surface disorder, preferably dry eye syndrome in a mammal.

The present invention furthermore relates to xanthohumol or a stereoisomer, tautomer, pharmaceutically acceptable salt, solvate or prodrug thereof for use in the treatment of a corneal surface disorder, preferably dry eye syndrome, or to diagnose, cure, mitigate, treat, or prevent a corneal surface disorder, preferably dry eye syndrome, in a mammal.

In addition, the present invention provides a method of treating, diagnosing, curing, mitigating or preventing a corneal surface disorder, preferably dry eye syndrome, comprising administering an effective amount of an ophthalmic composition according to the present invention to an eye of a mammal in need thereof.

The dry eye syndrome is preferably selected from lipid anomaly dry eye (LADE), aqueous tear deficiency (ATD), primary mucin anomalies, allergic/toxic dry eye (ADE), primary epitheliopathies and lid surfacing/blinking anomalies (LSADE). Diagnostic criteria for dry eye and dry eye subtypes may include the following:
LADE: Colored lipid interference fringes, or particulate, frothy or cloudy meibomian excreta dilated, plugged, capped, atrophied meibomian glands.
ATD: CTTT (cotton thread tear test) < 11 mm/15 s in either eye, and inferior tear meniscus < 0.1 mm in the same eye
Primary mucin anomaly: Conjunctival ciccatricial changes associated with:
   Stevens Johnson syndrome
   Ocular ciccatricial pemphigoid
   Pseudopemphigoid
   Chemical burns
   Trachoma scarring
ADE: Atopic history, and itch, and tarsal papillary response or conjunctival follicular drug toxicity response
Primary epitheliopathy: Neurotrophic, traumatic, degenerative and hereditary conditions affecting the corneal epithelium and basement membrane
LSADE: Anomalies of lid positioning, lid closure and/or blinking: ectropion, entropion, dermatochalasis, pterygium, pinguecula, incomplete blinking, proptosis, nocturnal lagophthalmos, blepharospasm, Bell's palsy.

It is to be understood that the ophthalmic composition or xanthohumol can be used before, during or after a surgical treatment of the eye, e.g. refractive surgeries, glaucoma surgeries.

Preferably, the ophthalmic composition or xanthohumol is to be used in ocular surface and corneal diseases, such as corneal erosion and keratitis punctata.

The mammal may be a human mammal or a non-human mammal, such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig. However, the mammal is preferably a human (e.g., a male human or a female human).

In the ophthalmic composition of the present invention, xanthohumol is preferably in the form of xanthohumol or a pharmaceutically acceptable salt or solvate thereof. Thus, the scope of the invention embraces all pharmaceutically acceptable salt forms of xanthohumol which may be formed, e.g. as a salt of an acid group (such as the phenolic group) with a physiologically acceptable cation. It is believed that xanthohumol has a first pkₐ of about 7 and may thus form salts in a pharmaceutically acceptable pH range. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts.

Moreover, the scope of the invention embraces xanthohumol in any solvated form, including, e.g., solvates with water, for example hydrates, or with organic solvents such as, e.g., methanol, ethanol or acetonitrile, i.e., as a methanolate, ethanolate or acetonitrilate, respectively, or in the form of any polymorph. It is to be understood that such solvates of xanthohumol also include solvates of pharmaceutically acceptable salts of xanthohumol.

Furthermore, xanthohumol may exist in the form of different isomers, in particular stereoisomers (in particular cis/trans isomers) or tautomers. All such isomers of xanthohumol are contemplated as being part of the present invention, either in admixture or in pure or substantially pure form. An example of an isomer or tautomer of xanthohumol includes isoxanthohumol ((S)-7-Hydroxy-2-(4-hydroxyphenyl)-5-methoxy-8-(3-methylbut-2-en-1-yl)chroman-4-one or (R)-7-Hydroxy-2-(4-hydroxyphenyl)-5-methoxy-8-(3-methylbut-2-en-1-yl)chroman-4-one, preferably (S)-7-Hydroxy-2-(4-hydroxyphenyl)-5-methoxy-8-(3-methylbut-2-en-1-yl)chroman-4-one). In the present invention, xanthohumol or isoxanthohumol or a mixture thereof may be used, with xanthohumol being preferred. It is also envisaged that 8-prenylnaringenin may be used instead of, or in combination with, xanthohumol.

The scope of the invention also embraces xanthohumol, in which one or more atoms are replaced by a specific isotope of the corresponding atom. For example, the invention encompasses xanthohumol, in which one or more hydrogen atoms (or, e.g., all hydrogen atoms) are replaced by deuterium atoms (i.e., ²H; also referred to as "D"). Accordingly, the invention also embraces xanthohumol which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in xanthohumol can be increased using deuteration techniques known in the art. For example, xanthohumol or a reactant or precursor to be used in the synthesis of xanthohumol can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that xanthohumol is not enriched in deuterium. Accordingly, the presence of naturally occurring hydrogen atoms or ¹H hydrogen atoms in xanthohumol is preferred.

Pharmaceutically acceptable prodrugs of xanthohumol are derivatives which have chemically or metabolically cleavable groups and become, by solvolysis or under physiological conditions, xanthohumol which are pharmaceutically active *in vivo.* Prodrugs of the compounds according to the the present invention may be formed in a conventional manner with a functional group of the compounds, in particular with one of the hydroxyl groups. The prodrug form often offers advantages in terms of solubility, tissue compatibility or delayed release in a mammalian organism (see, Bundgaard, H., Design of Prodrugs, pp. 7-9, 21-24, Elsevier, Amsterdam 1985). Prodrugs include an acyloxy derivative prepared by reacting one of the hydroxyl groups with a suitable acylhalide or a suitable acid anhydride. An especially preferred acyloxy derivative as a prodrug is -OC(=O)-CH₃, -OC(=O)-C₂H₅, -OC(=O)-(tert-Bu), -OC(=O)-C₁₅H₃₁, -OC(=O)-(m-COONa-Ph), -OC(=O)-CH₂CH₂COONa, -O(C=O)-CH(NH₂)CH₃ or -OC(=O)-CH₂-N(CH₃)₂.

The term "treatment" of a disorder or disease as used herein is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, inter alia, comprise curative treatment (preferably leading to a complete response and eventually to healing of the disorder or disease) and palliative treatment (including symptomatic relief).

The term "prevention" of a disorder or disease as used herein is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of the aqueous pharmaceutical composition of the present invention before any clinical and/or pathological symptoms are diagnosed or determined or can be diagnosed or determined by the attending physician.

The term "cure" of a disorder or disease as used herein refers to curative treatment, i.e. preferably leading to a complete response and eventually to healing of the disorder or disease.

The term "mitigate" of a disorder or disease as used herein is also well known in the art. For example, this refers to making the disorder or disease less harmful or unpleasant. In particular, it also refers to making pain or problems associated with the disorder or disease less severe.

The term "about" preferably refers to ±10% of the indicated numerical value, more preferably to ±5% of the indicated numerical value, and in particular, to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint.

The terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, if a component of a composition is indicated to be "optional", the invention specifically relates to both possibilities, i.e., that the corresponding component is present (contained in the composition) or that the corresponding component is absent from the composition.

The term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As used herein, unless explicitly indicated otherwise or contradicted by context, the terms "a", "an" and "the" are used interchangeably with "one or more" and "at least one". Thus, for example, a composition comprising "a" preservative can be interpreted as referring to a composition comprising "one or more" preservatives.

Unless specifically indicated otherwise, all properties and parameters referred to herein (including, e.g., any amounts/concentrations and any pH values) are preferably to be determined at standard ambient temperature and pressure conditions, particularly at a temperature of 25°C (298.15 K) and at an absolute pressure of 1 atm (101.325 kPa).

Moreover, unless indicated otherwise, any reference to an industry standard, a pharmacopeia, or a manufacturer's manual refers to the corresponding latest (i.e., most recent) version that was available on May 1, 2021.

It is to be understood that the present invention specifically relates to each and every combination of features and embodiments described herein, including any combination of general and/or preferred features/embodiments.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The present invention illustrated by the following examples which should not be construed as limiting.

### EXAMPLES

### METHODS

### Corneal Epithelial Cell Viability and Proliferation

Human corneal epithelial cells (hCEC; SCCE016; Merck Millipore, Burlington, MA, USA) were cultured in corneal epithelial cell growth medium with supplement mix (LGC Standards, Teddington, United Kingdom). Approximately 10,000 hCECs were seeded onto each well of a 96-well cell-culture dishes (NUNC, Langenselbold, Germany). Confluent cells were used for Water-soluble tetrazolium salt (WST-1) cell proliferation assays (Hoffmann-La Roche AG, Basel, Switzerland). After 24 hours of incubation with 100 µl of corneal epithelial cell growth medium without its supplement mix (LGC Standards, Teddington, United Kingdom), hCECs were treated with different concentrations of xanthohumol (XH; Selleckchem, Houston, TX, USA) dissolved in dimethyl sulfoxide (DMSO; Carl Roth AG, Karlsruhe, Germany). Control groups were treated with DMSO only. Following another 72 hours of incubation, the cells were incubated with the WST-1 solution for 30 minutes. Substrate turnover was measured with an ELISA reader at 450 nm (Spectramax 190; Molecular Devices, Sunnyvale, CA, USA) and a reference at 690 nm.

Proliferation of hCEC was determined using a 5-bromo-2'-deoxyuridine (BrdU) ELISA in accordance with the manufacturers' recommendations (Merck KGaA, Darmstadt, Germany). In brief, approximately 7,000 cells were seeded onto a 96-well cell culture plat e and incubated for 24h to achieve complete adherence of the cells. After 24 hours of incubation with 100 µl of corneal epithelial cell growth medium without its' supplement mix, hCECs were treated with xanthohumol dissolved in DMSO. Following another 24 hours of incubation with BrdU labeling solution, cells were fixed and incubated with anti-BrdU antibodies according to the manufacturer's recommendations. BrdU incorporation into the DNA of hCECs was quantified by absorbance measurement at 450 nm.

### Corneal Epithelial Cell Wound Healing

To analyze hCEC migration *in vitro,* cells were cultured in corneal epithelial cell growth medium with supplement mix until confluence of the cells of a 6-well cell culture plate. Following an incubation period of 24 hours in cell culture medium without supplements, 5 linear scratches were performed in each well using a 200 µl pipette tip (Eppendorf AG, Hamburg, Germany). The hCECs were then incubated prospectively under treatment with xanthohumol or DMSO as a control for up to 72 hours. Reference images were acquired at the beginning of the incubation period using the phase contrast mode on an Axio Observer 7 (Carl Zeiss AG, Jena, Germany). Every 24 hours, follow-up images of 4-5 different scratch sections were acquired. The size of the wound area was analyzed using a semi-automated analysis tool in ImageJ 1.53a (National Institute of Health, Bethesda, MD, USA).

Furthermore, corneal epithelial cell wound healing was determined on bovine corneas *ex vivo.* Bovine globes were obtained from a local butchery following approval by the authority for waste management of animal carcasses (ID: DE 09 162 0066-219) and processed within 12 hours *post-mortem.* The bovine globes were washed in 10% w/v povidone-iodine for 5 minutes. A circular corneal epithelial erosion was created using an 8 mm trephine and a hockey knife (Katena Products Inc., Parsippany, NJ, USA). Subsequently, a sclerocorneal ring was excised with eye scissors (Katena Products Inc., Parsippany, NJ, USA) and transferred to a 60 mm cell culture plate. To acquire reference images of the corneal erosion, the wound area was stained with a 0.8 mg/ml fluorescein sodium solution and illuminated with blue light (approximate wavelength 490 nm). Images were acquired using a distance-fixed digital single lens reflex camera equipped with a macro lens (Canon Inc., Tokyo, Japan). Consequently, the sclerocorneal rings were incubated in Dulbecco's Modified Eagle's Medium (Bio&SELL GmbH, Feucht, Germany) supplemented with 50 IU penicillin/ml and 50 µg streptomycin/ml (Merck Millipore, Burlington, MA, USA) and xanthohumol or DMSO only. Follow-up images were acquired every 12 hours, the cell culture medium was renewed daily.

### Oxidative Stress Models for Corneal Epithelial Cells

To determine potential anti-oxidative effects of xanthohumol, hCECs were incubated and treated analogous to the above-mentioned WST-1 cell proliferation assay with minor modifications. To induce oxidative stress, hydrogen peroxide (H₂O₂) in a concentration of 100 µM was added to the treatment groups and an additional H₂O₂-only group was tested. Following another incubation of the hCECs for 24 hours, the WST-1 assay was further finalized according to the description above.

Intracellular, mitochondrial reactive oxygen species (ROS) were assessed using the cell membrane permeable dihydrorhodamine-123 (DHR-123) assay (D23806; ThermoFisher Scientific, Waltham, MA, USA) according to the manufacturer's instructions. Measurements were made after exposing human corneal epithelial cells to H₂O₂, xanthohumol or DMSO only for 2 hours. For ROS staining, cells were incubated with 10 µM DHR-123 for 15 minutes. Nucleus staining was performed using Hoechst 33342 nucleic acid stain (H1399; ThermoFisher Scientific, Waltham, MA, USA). Randomly, a minimum of 3 images of every treatment group were acquired with a 20x fluorescence objective using the aforementioned Axio Observer 7. Results were expressed in percent of fluorescence intensity relative to the baseline measurement in the DMSO control group. For quantification of the fluorescence signals, the microscope software ZEN 3.0 (Carl Zeiss AG, Jena, Germany) was used.

### Data Management and Statistical Analysis

All results are expressed as mean ± standard deviation. A one-way analysis of variance (ANOVA) was performed to compare the mean variables of more than 2 groups. A Bonferroni post-hoc test followed for data that meet or do not meet the criteria of the assumption of homogeneity of variances, respectively. To depict significant changes over time (wound healing assays) a two-way ANOVA with Geisser-Greenhouse correction was conducted. P-values less than 0.05 were considered to be statistically significant.

### RESULTS

### Xanthohumol Improves Corneal Epithelial Cell Viability

In response to 72 hours of incubation with 1 µM and 2.5 µM xanthohumol, hCECs showed a significant increase of cell viability to 144 ± 26% and 127 ± 11%, respectively, when compared to the DMSO-treated control group (p < 0.0001 and p = 00335; Fig. 1). The treatment with 5 µM xanthohumol did not show significant changes of hCEC viability (121 ± 24%, p = 0.2234; Fig. 1).

### Xanthohumol Does Not Decrease Corneal Epithelial Cell Proliferation

Figure 2 indicates that after an incubation of hCECs with xanthohumol for 24 hours, no significant changes of cell proliferation at a concentration of 1 µM (112 ± 26%), 2.5 µM (107 ± 16%) nor at 5 µM (95 ± 8%) were detected (p > 0.9999 for all groups). However, a pro-proliferative tendency at a concentration of 1 µM and 2.5 µM xanthohumol could be observed (Fig. 2).

### Xanthohumol Enhances Corneal Epithelial Cell Migration In Vitro

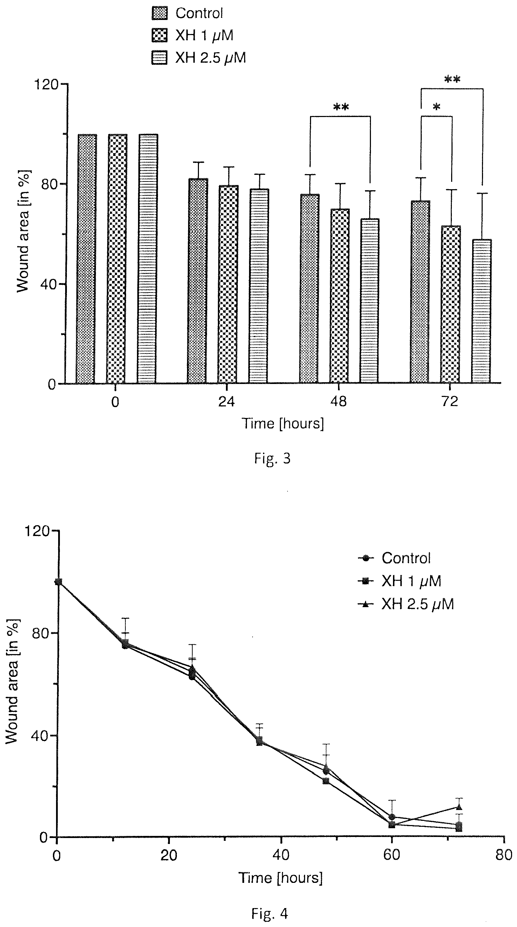

Figure 3 displays the scratched hCEC area over time when treated with either xanthohumol or astragaloside IV in percent of the corresponding DMSO-only control group. After 48 hours, a significantly smaller wound area of 66 ± 11% was observed at a xanthohumol concentration of 2.5 µM compared to the control group (76 ± 7%, p = 0.004). Following 72 hours of incubation with xanthohumol at a concentration of 1 µM and 2.5 µM, a significant reduction of the initial wound area to 63 ± 14% (p = 0.0352) and 58 ± 18% (p = 0.0052), respectively, was observed (Fig. 3). The corresponding control group showed a residual wound area of 73 ± 9% after 72 hours (Fig. 3). Besides that, no significant differences between the remaining treatment groups could be observed (p > 0.05 for all groups; Fig. 3).

### No Antimigratory Effects of Xanthohumol Ex Vivo

Corneal epithelial wound healing was analyzed for up to 72 hours until subtotal re-epithelialization of the initial surface defect (Fig. 4). When treated with 1 µM or 2.5 µM of xanthohumol, bovine corneas did not show any significant difference in the speed of re-epithelialization in comparison to the DMSO-treated control group. This effect was present at all measurement points (every 12 hours) and for both concentrations of XH (p > 0.05 for all groups).

### Xanthohumol Protects Corneal Epithelial Cells from Oxidative Stress

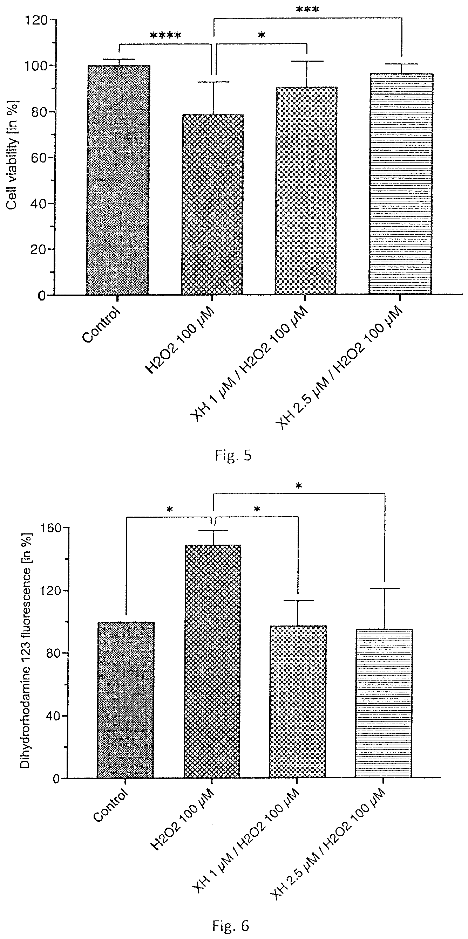

When exposed to H₂O₂ for 24 hours, cell viability of human corneal epithelial cells was significantly reduced to 79 ± 14% compared to the untreated control group (100 ± 3%, p < 0.0001; Fig. 5). Interestingly, this effect could be neutralized when xanthohumol was present in a concentration of 1 µM or 2.5 µM. Corneal epithelial cell viability in those groups accounted for 91 ± 11% and 96 ± 4%, respectively (Fig. 5). In comparison to the H₂O₂ group, those values are significantly higher (p = 0.0110 and p = 0.0001) while no significant difference compared to the untreated control group could be observed (p = 0.0551 and p > 0.9999; Fig. 5).

Intracellular reactive oxygen species levels were assessed by measuring the fluorescence intensity of dihydrorhodamine 123 after incubation with DMSO, H₂O₂ or the combination of the latter with xanthohumol. The exposure of hCECs to H₂O₂ led to a significant increase of DHE-123 fluorescence to 149 ± 9% compared to the control group (p = 0.0301; Fig. 6). In the presence of xanthohumol in a concentration of 1 µM and 2.5 µM DHR-123 fluorescence was 97 ± 16% and 95 ± 26%, respectively, and thus significantly lower than the H₂O₂ group (p = 0.0227 and p = 0.0178; Fig. 6). No significant differences between the control group and xanthohumol-supplemented groups were observed (p > 0.9999 for both groups).

From these data, it can be concluded that xanthohumol or a stereoisomer, tautomer, pharmaceutically acceptable salt, solvate or prodrug thereof may be used in the treatment of a corneal surface disorder, preferably dry eye syndrome, or to diagnose, cure, mitigate, treat, or prevent a corneal surface disorder, preferably dry eye syndrome, in a mammal.

## Claims

1. An ophthalmic composition comprising xanthohumol or a stereoisomer, tautomer, pharmaceutically acceptable salt, solvate or prodrug thereof.

2. The ophthalmic composition according to claim 1, wherein the concentration of xanthohumol or a stereoisomer, tautomer, pharmaceutically acceptable salt, solvate or prodrug thereof in the ophthalmic composition is in a range of from 0.001 to 100 µM, preferably 0.01 to 20 µM, more preferably 0.1 to 10 µM, even more preferably 0.2 to 10 µM, still more preferably 0.5 to 6 µM, most preferably 1 to 3 µM, based on the total volume of the ophthalmic composition.

3. The ophthalmic composition according to claim 1 or 2, wherein the ophthalmic composition is an aqueous composition.

4. The ophthalmic composition according to claim 3, wherein the ophthalmic composition is a solution or an emulsion.

5. The ophthalmic composition according to any one of claims 1 to 4, wherein the ophthalmic composition is an oil-in-water emulsion.

6. The ophthalmic composition according to any one of claims 1 to 5, wherein the ophthalmic composition further comprises one or more antioxidants, wherein the one or more antioxidants preferably include one or more selected from sodium metabisulfite, sodium thiosulfate, acetylcysteine, butylated hydroxyanisole, and butylated hydroxytoluene.

7. The ophthalmic composition according to claim 6, wherein the ophthalmic composition comprises the one or more antioxidants at a concentration in a range of from 0.0001 wt.-% to 5 wt.-%, preferably from 0.2% to 5 wt.-%, more preferably from 0.5 wt.-% to 2 wt.-%, based on the total weight of the ophthalmic composition.

8. The ophthalmic composition according to any one of claims 1 to 7, wherein the ophthalmic composition is for use in the treatment of a corneal surface disorder, preferably dry eye syndrome, or to diagnose, cure, mitigate, treat, or prevent a corneal surface disorder, preferably dry eye syndrome, in a mammal.

9. Xanthohumol or a stereoisomer, tautomer, pharmaceutically acceptable salt, solvate or prodrug thereof for use in the treatment of a corneal surface disorder, preferably dry eye syndrome, or to diagnose, cure, mitigate, treat, or prevent a corneal surface disorder, preferably dry eye syndrome, in a mammal.

10. A method of treating, diagnosing, curing, mitigating or preventing a corneal surface disorder, preferably dry eye syndrome, comprising administering an effective amount of an ophthalmic composition according to any one of claims 1 to 8 to an eye of a mammal in need thereof.

11. The ophthalmic composition for use according to claim 8, xanthohumol for use according to claim 9 or the method according to claim 10, wherein the mammal is a human.

12. The ophthalmic composition for use according to claim 8 or 11, xanthohumol for use according to claim 9 or 11 or the method according to claim 10 or 11, wherein xanthohumol is in the form of xanthohumol or a pharmaceutically acceptable salt or solvate thereof.

13. The ophthalmic composition for use according to claims 8, 11 and 12, xanthohumol for use according to claims 9, 11 and 12 or the method according to claims 10, 11 and 12, wherein the dry eye syndrome is lipid anomaly dry eye (LADE), aqueous tear deficiency (ATD), primary mucin anomalies, allergic/toxic dry eye (ADE), primary epitheliopathies and lid surfacing/blinking anomalies (LSADE).

14. The ophthalmic composition for use according to claims 8, 11, 12 and 13, xanthohumol for use according to claims 9, 11, 12 and 13 or the method according to claims 10, 11, 12 and 13, wherein the ophthalmic composition or xanthohumol is to be used before, during or after a surgical treatment of the eye, e.g. refractive surgeries, glaucoma surgeries.

15. The ophthalmic composition for use according to claims 8, 11, 12 and 13, xanthohumol for use according to claims 9, 11, 12 and 13 or the method according to claims 10, 11, 12 and 13 wherein the ophthalmic composition or xanthohumol is to be used in ocular surface and corneal diseases, such as corneal erosion and keratitis punctata.
